# EUROPEAN PATENT APPLICATION

(11) **EP 4 691 370 A2**
(43) Date of publication of application: **11.02.2026**
(21) Application number: 24781105.2
(22) Date of filing: 20.03.2024
(51) Int. Cl.: A61B 6/00, A61B 6/04

(54) **X-RAY IMAGING DEVICE FOR ANIMALS**

(30) Priority: 27.03.2023 KR 20230039628
(71) Applicant: Tuavet Inc., Hwaseong-si, Gyeonggi-do 18559 (KR)
(72) Inventor: LEE, Jaseong, Seoul 03944 (KR)
(74) Representative: Ström & Gulliksson AB
(86) International application number: PCT/KR2024/003477
(87) International publication number: WO 2024/205114

(57) **Abstract**

The present invention relates to an X-ray imaging apparatus 100 for animals. An X-ray tube 121 and an X-ray detector 123 of the present invention may provide optimal imaging results desired by a veterinarian. Through a dynamic mechanism of a table assembly system, the X-ray imaging apparatus may effectively perform general imaging for 2D images, 3D rotational imaging, and standing imaging with only a single X-ray imaging device.

## Description

### TECHNICAL FIELD

The present invention relates to an X-ray imaging apparatus for animals.

### BACKGROUND ART

An X-ray imaging apparatus is utilized for accurate diagnosis and treatment of animals. In veterinary clinics, the subject of an X-ray imaging apparatus is an animal, and more specifically, a small animal such as a dog or a cat. When the subject is a human, communication between an operator and the subject is possible. Accordingly, the operator can easily obtain a desired image using the apparatus. However, when the subject is an animal, such communication is not possible. Thus, there are various difficulties, such as the operator needing to physically hold the subject during imaging.

Meanwhile, various types of X-ray imaging apparatuses have become known depending on their respective purposes. For example, an apparatus intended for acquiring a 2D image differs in shape and structure from one intended for acquiring a 3D image. These two types of apparatus are generally manufactured and used separately. Since the latter is more expensive than the former, such equipment is mainly used in hospitals that provide advanced care. Hospitals for humans are equipped with multiple types of X-ray imaging apparatuses depending on the level of their diagnostic systems, ranging from private clinics to general hospitals. Patients can choose a hospital based on the affected part and the severity of the disease without much difficulty. However, in the case of animals, access to hospitals is not the same as for humans. Above all, due to the cost burden and spatial limitations in veterinary clinics, it has been difficult to equip multiple devices configured for their respective purposes. In addition, when the subject is a small animal, imaging diagnosis and treatment must often be performed in the same location, and thus, conventional human equipment cannot adequately perform such needs.

The inventor of the present invention has devoted much effort and research to address the above-described problems and has completed the present invention as a result.

### DISCLOSURE OF THE INVENTION

### TECHNICAL PROBLEM

It is an object of the present invention to provide a system apparatus for diagnosing small animals in veterinary clinics. More specifically, the present invention aims to provide a single X-ray imaging apparatus that may perform general imaging, 3D imaging, and standing imaging in a flexible manner. Through this, the present invention may provide a diagnostic system that may perform the above-mentioned multiple types of X-ray imaging required for the diagnosis and treatment of small animals-such as simple diagnosis, advanced treatment, image acquisition for various parts of a small animal, and real-time diagnosis and treatment-using a single apparatus.

As a result, another object of the present invention is to contribute to the reduction of costs in veterinary clinics while also contributing to the advancement of veterinary medicine and the improvement of pet health.

Meanwhile, other unspecified objects of the present invention may also be additionally considered to the extent that they can be readily inferred from the following detailed description and the effects thereof.

### TECHNICAL SOLUTION

The present invention provides an X-ray imaging apparatus for animals including: a main body; an arm operating part structured such that an X-ray tube and an X-ray detector are arranged in a line facing each other; an arm rotating part configured to connect the arm operating part to the main body and to rotate; and a table connected to the main body and on which a subject is to be positioned, the table including a center table assembly and displacement table assemblies connected to both sides of the center table assembly, respectively, wherein, in a first imaging mode, the displacement table assemblies are located on left and right sides in a longitudinal direction of the center table assembly, respectively, so as to secure a reference position for an operator, and in a second imaging mode, a first table rotating means, which connects the center table assembly and a first displacement table assembly of the displacement table assemblies, operates to move the first displacement table assembly to a front-left side in a width direction of the center table assembly, and a second table rotating means, which connects the center table assembly and a second displacement table assembly of the displacement table assemblies, operates to move the second displacement table assembly to a front-right side in the width direction of the center table assembly, such that rotation of the arm operating part is ensured.

The present invention also provides an X-ray imaging apparatus for animals including: a main body; an arm operating part structured such that an X-ray tube and an X-ray detector are arranged in a line facing each other; an arm rotating part configured to connect the arm operating part to the main body and to rotate; and a table connected to the main body and on which a subject is to be positioned, the table including a center table assembly and displacement table assemblies connected to both sides of the center table assembly, respectively, wherein, in a first imaging mode, the displacement table assemblies are located on left and right sides in a longitudinal direction of the center table assembly, respectively, so as to secure a reference position for an operator, and in a second imaging mode, a first table rotating means, which connects the center table assembly and a first displacement table assembly of the displacement table assemblies, operates to move the first displacement table assembly to a front-left side in a width direction of the center table assembly, and a second table rotating means, which connects the center table assembly and a second displacement table assembly of the displacement table assemblies, operates to move the second displacement table assembly to a front-right side in the width direction of the center table assembly, so as to ensure rotation of the arm operating part, and in a third imaging mode, the arm rotating part is displaced downward from the main body, and among the connectors connecting the center table assembly to the main body, a connector that is detachably coupled is detached, and the center table assembly is eccentrically rotated via a rotating hinge provided at an end of the other connector, so that the center table assembly is spaced apart from the main body to the maximum extent, thereby ensuring horizontal imaging at a lowest position level by the arm operating part.

### ADVANTAGEOUS EFFECTS

According to the present invention, even if only the single X-ray imaging apparatus is operated in the veterinary clinic, general imaging for acquiring 2D images, three-dimensional rotational imaging, and standing imaging may all be effectively performed. In the related art, a standing imaging apparatus required a separate X-ray detector stand, and there was a problem in that a large imaging space was needed.

In addition, according to the present invention, the space limitation in the veterinary clinic may be resolved and the economic burden may be reduced. From the standpoint of medical personnel, since the equipment necessary for advanced treatment may be easily used, better animal medical services may be provided.

Meanwhile, even effects that are not explicitly described herein may be regarded as being included in the effects of the present invention to the extent that they are expected based on the technical features of the present invention and described in the following specification.

### BRIEF DESCRIPTION OF THE DRAWINGS

FIG. 1 schematically illustrates a configuration of an X-ray imaging apparatus 100 for animals according to a preferred embodiment of the present invention.
FIGS. 2 and 3 illustrate a configuration and an example of use of an X-ray imaging apparatus in a first imaging mode according to an embodiment of the present invention.
FIGS. 4 and 5 illustrate a configuration and an example of use of the X-ray imaging apparatus in a second imaging mode according to an embodiment of the present invention.
FIGS. 6 and 7 illustrate a configuration and an example of use of the X-ray imaging apparatus in a third imaging mode according to an embodiment of the present invention.
FIG. 8 schematically illustrates a configuration of a central table assembly 200 according to a preferred embodiment of the present invention.
FIG. 9 schematically illustrates a configuration of displacement table assemblies 300 and 400 according to a preferred embodiment of the present invention.
FIG. 10 schematically illustrates a state in which the central table assembly 200 of FIG. 8 and the displacement table assemblies 300 and 400 of FIG. 9 are coupled to each other for the first imaging mode.
FIGS. 11 to 13 illustrate coupling relationships of lower components of the table assemblies 200, 300, and 400 according to a preferred embodiment of the present invention.
FIG. 14 illustrates a process of displacement from the first imaging mode to the second imaging mode according to an embodiment of the present invention.
FIG. 15 illustrates a process of displacement from the first imaging mode to the second imaging mode according to another embodiment of the present invention.

The attached drawings are provided by way of reference to facilitate understanding of the technical spirit of the present invention, and the scope of the present invention shall not be construed as being limited thereto.

### MODE FOR CARRYING OUT THE INVENTION

Hereinafter, specific details of the present invention will be described with reference to the drawings, which illustratively show a configuration of the present invention. Moreover, detailed descriptions related to well-functions or configurations will be ruled out in order not to unnecessarily obscure subject matters of the present invention.

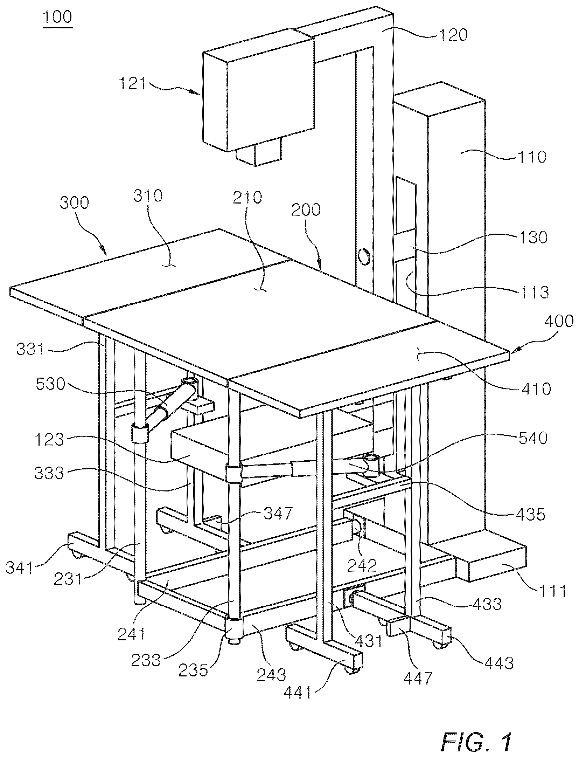

FIG. 1 schematically illustrates the entire configuration of an X-ray imaging apparatus 100 for animals according to a preferred embodiment of the present invention.

An X-ray imaging apparatus 100 for animals according to the present invention may include a main body 110, an arm operating part 120, an arm rotating part 130, a central table assembly 200, and a pair of displacement table assemblies 300 and 400.

An X-ray tube 121 may be provided at one end of the arm operating part 120, and an X-ray detector 123 may be provided at the other end of the arm operating part 120. The X-ray tube 121 and the X-ray detector 123 may be configured to face each other and be aligned in a straight line. The X-ray tube 121 may irradiate radiation toward a subject. The X-ray detector 123 may receive the radiation irradiated from the opposing X-ray tube 121. A light irradiation mechanism of the X-ray tube 121 and a light receiving mechanism of the X-ray detector 123 may include a shutter, a control unit, a monitor, a communication component, and a power component. Since these components are widely used known technologies in X-ray imaging apparatuses, a detailed description thereof will be omitted.

One end of a cylindrical arm rotating part 130 may be connected to the arm operating part 120, and the other end of the arm rotating part 130 may be provided on an elevating part 113 of the main body 110. A motor and mechanical components may be embedded in the arm rotating part 130. Due to their mechanical operations, the arm rotating part 130 may ascend or descend through the elevating part 113 of the main body 110 and may also rotate about 360-degree range. As the arm rotating part 130 rotates, the arm operating part 120 may rotate accordingly, and as the arm rotating part 130 ascends or descends, the arm operating part 120 may also be vertically displaced. As a result, an X-ray imaging mode executed by the X-ray tube 121 and the X-ray detector 123 may dynamically change. However, as shown in the illustrated example, the mechanical movement of the arm operating part 120 may inevitably be obstructed by the positions of the central table assembly 200 and the displacement table assemblies 300 and 400. The X-ray imaging apparatus 100 for animals according to the present invention may flexibly respond to and resolve such collision issues. Its mechanical solution will be disclosed through this specification.

A subject 10 may be disposed on a central table 210 of the central table assembly 200, a first displacement table 310 of a first displacement table assembly 300, and a second displacement table 410 of a second displacement table assembly 400. In a preferred embodiment of the present invention, these tables may be made of the same material. In another preferred embodiment of the present invention, the central table 210 may be made of a plate made of an X-ray transmissive material, and the first displacement table 310 and the second displacement table 410 may be made of plates coated with an X-ray shielding material. This is because the X-ray tube 121 may be positioned above (or below) the central table 210 in one direction, and the X-ray detector 123 may be positioned below (or above) in the other direction. In the illustrated example, the central table 210, the first displacement table 310, and the second displacement table 410 are aligned in a longitudinal direction.

Preferably, a pair of main body connectors 241 and 243 of the central table assembly 200 may be provided by being connected to a lower portion 111 of the main body 110, whereas the first displacement table assembly 300 and the second displacement table assembly 400 may remain independent of the main body 110. This may serve as one element of mechanical flexibility of the X-ray imaging apparatus 100 for animals according to the present invention. In a preferred embodiment, an end of a first main body connector 241 may be provided as a detachable member 242 to be coupled to or separated from the lower portion 111 of the main body. In contrast, a second main body connector 243 is preferably fixedly coupled to the lower portion 111 of the main body. The effect resulting from the difference in the connection manner between the first main body connector 241 and the second main body connector 243 to the lower portion 111 of the main body will be described later.

The central table assembly 200 and the displacement table assemblies 300 and 400 may have the same height. Preferably, casters may be provided on the floor-contacting ends of the assemblies 200, 300, and 400.

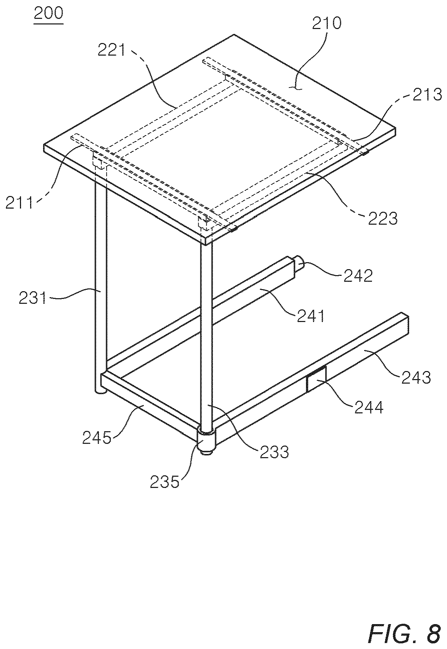

FIG. 8 schematically illustrates a configuration of the central table assembly 200 according to a preferred embodiment of the present invention. A first vertical support 231 and a second vertical support 233 may be respectively connected to the aforementioned main body connectors 241 and 243. Lower ends of the first vertical support 231 and the second vertical support 233 may be connected to each other by a horizontal bar 245. The first vertical support 231 and the second vertical support 233 may reach a predetermined height of the central table assembly 200 and may be connected to a first table support 221 and a second table support 223, respectively. Linear motion guide members 211 and 213 may be provided on the table supports 221 and 223 to support a central table 210, and may assist in a slight displacement of the central table 210 in a longitudinal and lateral direction. The central table 210 may be mounted on the linear motion guide members 211 and 213. The linear motion guide members 211 and 213 are commonly used means in the field for facilitating collimation, which is the process of determining an X-ray imaging region of a subject.

A rotating hinge 235 may be provided at a lower end of the second vertical support 233. The rotating hinge 235 may allow the entire central table assembly 200 to eccentrically rotate counterclockwise around the second vertical support 233, in a state where the second main body connector 243 remains is fixed to the main body 110.

Detachable members 244 and 244 may be respectively provided on the sides of the main body connectors 241 and 243 facing the displacement table assemblies 300 and 400, so as to be detachable from base ends of the displacement table assemblies 300 and 400. Preferably, a magnetic holding coupling may be employed.

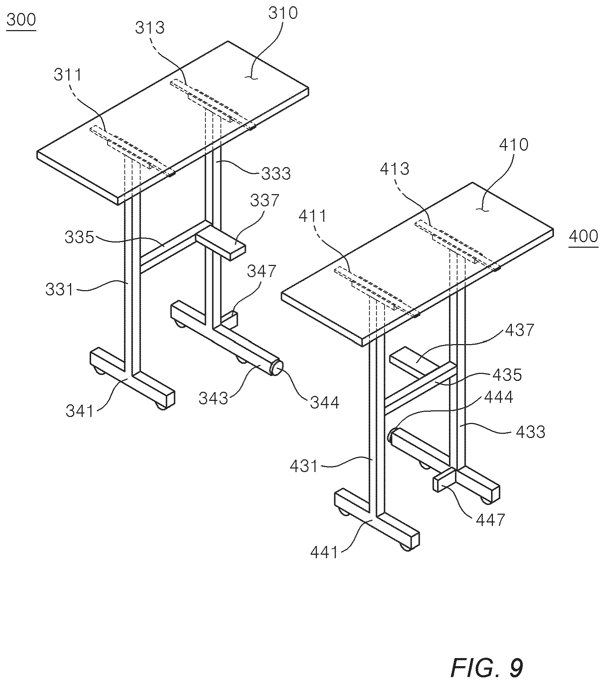

FIG. 9 schematically illustrates a configuration example of the displacement table assemblies 300 and 400 according to a preferred embodiment of the present invention.

In the embodiment of FIG. 1, the first displacement table assembly 300 may be disposed on a left side of the aforementioned central table assembly 200. A first vertical support 331 and a second vertical support 333 may support the first displacement table 310. A first base 341 and a second base 343, which are respectively connected to the first vertical support 331 and the second vertical support 333, may be in contact with a floor surface through casters and may support the first displacement table assembly 300. A detachable member 344 may be provided on one end of the second base 343 to be detachable from a corresponding component of the central table assembly 200 or a corresponding means 447 of the second displacement table assembly 400. In addition, the corresponding means 347 may be provided on the second base 343 to be coupled to a detachable member 444 of a second base 443 of the second displacement table assembly 400.

As in the structure of the central table 210, linear motion guide members 311 and 313 may be provided on a lower portion of the second displacement table 310 to support the table and assist in slight displacement in longitudinal and lateral directions. The first displacement table 310 may be disposed on the linear motion guide members 311 and 313.

A horizontal bar 335 may connect and fix the first vertical support 331 and the second vertical support 333 to each other. A hinge end of the first table rotating arm may be provided on a hinge support 337 adjacent to the second vertical support 333 of the horizontal bar 335.

In the embodiment of FIG. 1, the second displacement table assembly 400 may be disposed on a right side of the aforementioned central table assembly 200. The first vertical support 431 and the second vertical support 433 may support the second displacement table 410, and a first base 441 and a second base 443, which are respectively connected to the vertical supports 431 and 433, may be in contact with a floor surface via casters and may support the second displacement table assembly 400. A detachable member 444 may be provided on one end of the second base 443 so as to be detachable from a corresponding component of the central table assembly 200 or a corresponding means 347 of the first displacement table assembly 300. In addition, a corresponding means 447 may be provided on the second base 443 to be coupled to a detachable member 344 of a first base 343 of the first displacement table assembly 300.

As in the structure of the first displacement table 310 and the central table 210, linear motion guide members 411 and 413 may be disposed at a lower portion of the second displacement table 410 to support the table and assist in slight displacement in longitudinal and lateral directions. The second displacement table 410 may be disposed on the linear motion guide members 411 and 413.

A horizontal bar 435 may connect and fix the first vertical support 431 and the second vertical support 433 to each other. A hinge end of the second table rotating arm may be provided on the hinge support 437 adjacent to the second vertical support 433 of the horizontal bar 435.

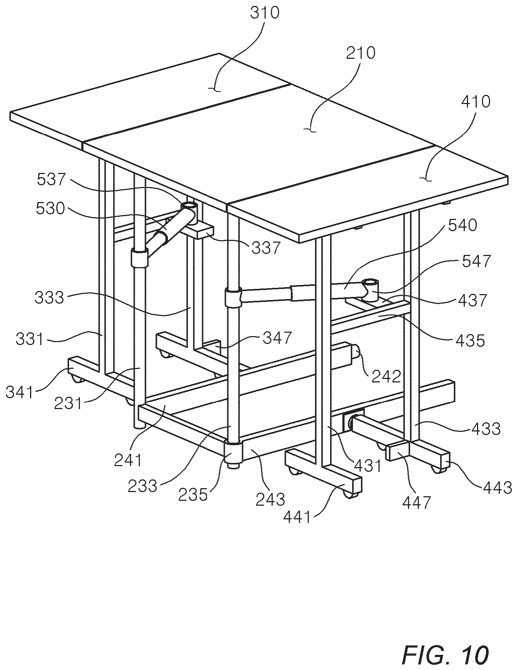

How, then, are the displacement systems of the first displacement table assembly 300 and the second displacement table assembly 400 configured? This will be explained with reference to the embodiment of FIG. 10.

FIG. 10 schematically illustrates a configuration example of a first table rotating arm 530 and a second table rotating arm 540 according to a preferred embodiment of the present invention.

The first table rotating arm 530 may connect the first vertical support 231 of the central table assembly 200 and the hinge support 337 of the first displacement table assembly 300 to each other. Hinges may be provided at both ends of the first table rotating arm 530. A hinge 531 at one end of the first table rotating arm 530 may be provided on the first vertical support 231, and another hinge 537 at the other end of the first table rotating arm 530 may be provided on the hinge support 337 of the first displacement table assembly 300. Preferably, a structure that allows the connection to be released may further be provided on the body of the first table rotating arm 530. Since the first table rotating arm 530 is separated into two parts, the first displacement table assembly 300 may be detached from the central table assembly 200.

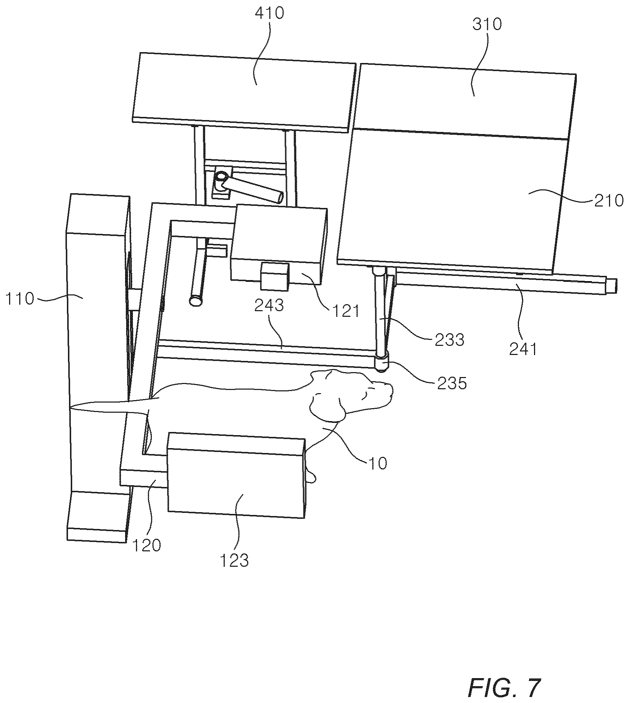

The second table rotating arm 540 may connect the second vertical support 233 of the central table assembly 200 and the hinge support 437 of the second displacement table assembly 400. Hinges may be provided on both ends of the second table rotating arm 540. A hinge 541 at one end of the second table rotating arm 540 may be provided on the second vertical support 233, and a hinge 547 at the other end of the second table rotating arm 540 may be provided on the hinge support 437 of the second displacement table assembly 400. Preferably, a structure that allows the connection to be released by separating the body of the second table rotating arm 540 into two parts may further be provided on the second table rotating arm 540, which may enable a third imaging mode, as will be described below, As shown in FIG. 7.

Referring again FIG. 1, in the embodiment illustrated in FIG. 10 of the present invention, the displacement table assemblies 300 and 400 may be connected to the central table assembly 200 in left and right directions along the longitudinal axis. At the same time, the central table assembly 200 may be connected to the main body 110, thereby configuring the X-ray imaging apparatus 100 for animals. The X-ray imaging apparatus 100 for animals according to the present invention may perform three imaging modes. The three imaging modes of the present invention are defined as follows.

A first imaging mode may be an X-ray imaging method for acquiring two-dimensional planar images. As shown in FIG. 1, an arm operating part 120 may be in a reference position. At this point, an X-ray tube 121 may be located above a center table assembly 200, and an X-ray detector 123 may be located below the center table assembly 200, it may be in a mode in which vertical X-ray imaging in the up-and-down direction is performed (the positions of the X-ray tube 121 and the X-ray detector 123 may be reversed). The embodiments of FIGS. 1 to 3 schematically illustrate the table system in the first imaging mode. In the first imaging mode, an operator may be positioned to face the main body 110 and the table assembly may be disposed between the operator and the main body 110 (operator reference position). The operator may refer to a virtual device user who holds the subject and performs imaging or treatment. During actual imaging, since two operators may hold a subject along its longitudinal direction, the operator may move away from the "O" position to move to the left or right side when operating the X-ray imaging apparatus.

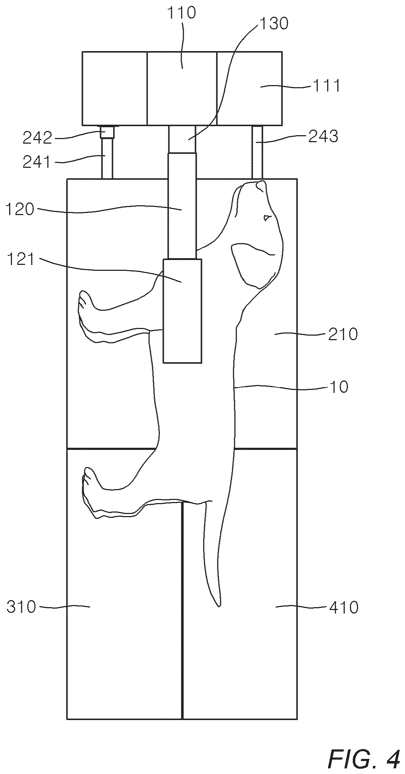

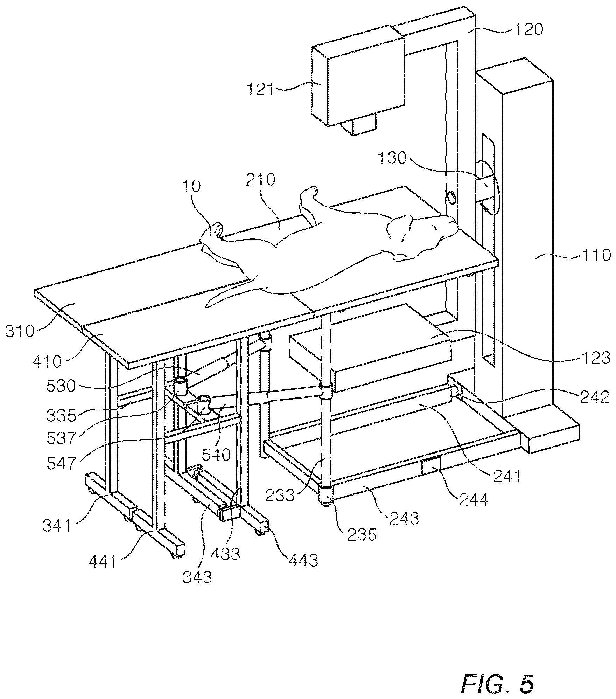

A second imaging mode may be an X-ray imaging method for acquiring a three-dimensional image necessary for three-dimensional diagnosis of the body of a small animal. An arm rotating part 130 may operate, and the arm operating part 120 may be positioned at the desired location of the operator within about 360-degree range. However, in the first imaging mode, since the first displacement table 310 and the second displacement table 410 are arranged along the left and right longitudinal sides of the center table 210, and a support system is positioned below the table, it may be difficult for the arm operating part 120 to rotate. Therefore, the first displacement table assembly 300 and the second displacement table assembly 400 may be rotated from the positions in the first imaging mode and may be displaced forward in the width direction of the center table assembly 200, so that the rotation of the arm operating part 120 may be ensured. Thus, integration of the general imaging mode (the first imaging mode) and the 3D rotational imaging mode (the second imaging mode), which has been difficult in the prior art, may be achieved. In addition, since the table is elongated in the width direction, even a dog breed with a relatively long body length-which may have been problematic in conventional 3D rotational imaging-may be sufficiently supported. The embodiments of FIGS. 4 and 5 may schematically illustrate the system in the second imaging mode.

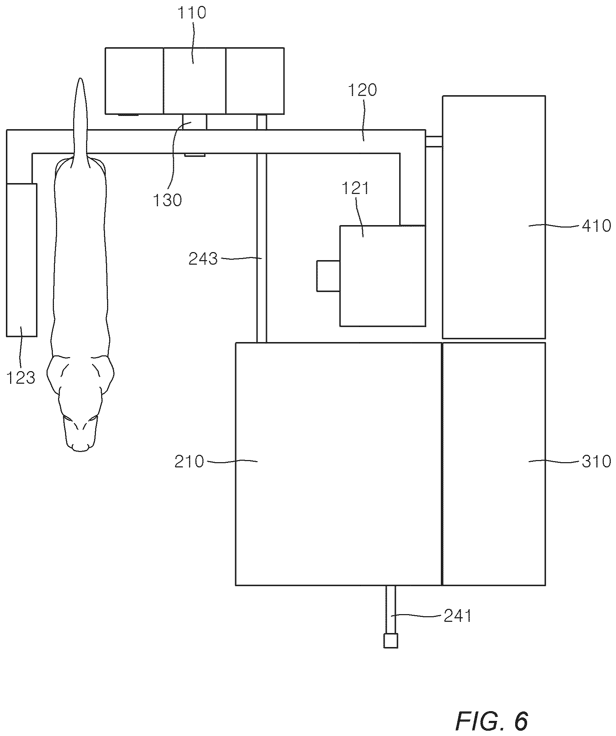

A third imaging mode may be a mode in which X-ray imaging may be performed with a subject in a standing position. For small animals, all of these imaging modes may be essential for swallowing and gait tests. In the prior art, a separate X-ray detector stand may have been required. However, such a stand may not be necessary in the present invention. In this mode, an arm rotating part 130 may be downwardly displaced from a main body elevating part 113, thereby ensuring that horizontal imaging may be performed at the lowest position level (horizontal imaging may be performed, or a slight tilt may be applied). The table assembly system of the present invention may not interfere with the low-level alignment of the arm operating part 120. The embodiments of FIGS. 6 and 7 may schematically illustrate the system in the third imaging mode.

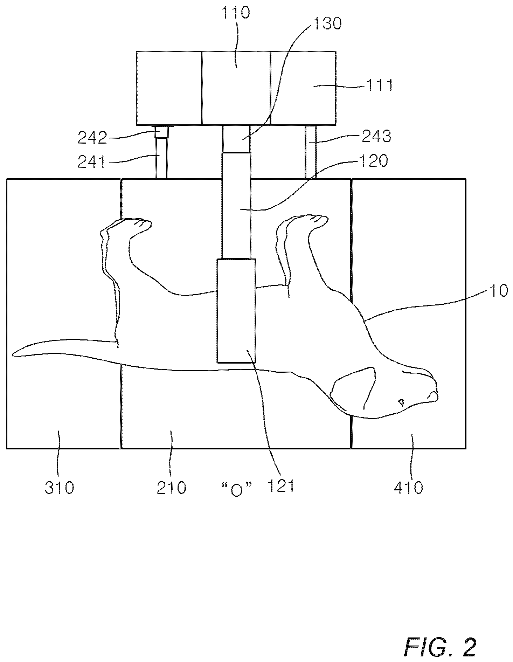

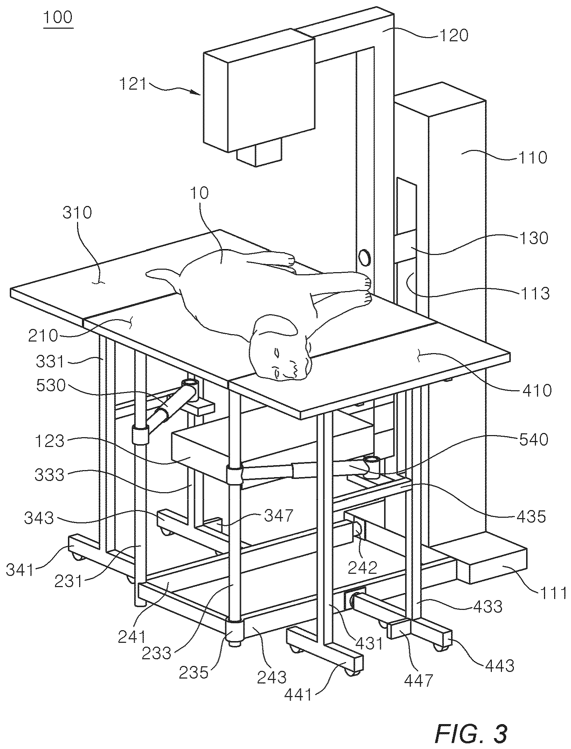

Now, the mechanism of the X-ray imaging apparatus of the present invention for each of these imaging modes may be described in detail. FIGS. 2 and 3 may illustrate embodiments related to the first imaging mode of the present invention.

Since the first displacement table 310 and the second displacement table 410 may be connected to both sides of the center table 210, the total length of the table may be extended. That is, the displacement tables 310 and 410 may respectively extend from the left and right sides of the center table 210 in the longitudinal direction. A subject 10 may be positioned on the extended table. For ease of understanding by those skilled in the art, the subject 10 may be illustrated larger than actual. Small dog breeds and most cats may have a size smaller than that of illustration of the subject 10. A reference position of the operator who may hold the subject 10 may be indicated as "O". The shortest straight-line direction between the operator's reference position "O" and the main body 110 may be referred to as a width direction. Currently, the subject 10 may lie in the longitudinal direction.

As described above, the first displacement table assembly 300 and the second displacement table assembly 400 may not be connected to the main body 110. However, the first displacement table assembly and the second displacement table assembly may be coupled to the first main body connector 241 and the second main body connector 243, respectively, by detachable members (e.g., magnetic members) of the second support 343 of the first displacement table assembly and the second support 443 of the second displacement table assembly. The first body connector 241 and the second body connector 243 of the center table assembly 200 may be connected to the main body 110. In this state in which the center table assembly is coupled to the main body 110, three table assembly components may be connected to each other under the table system to secure structural stability. Due to this structural stability, the operator may stably support even a vigorously moving subject 10 to perform general imaging. In addition, during general imaging, when the displacement tables 310 and 410 are not fixed, a gap may occur between the displacement tables 310, 410 and the center table 210 due to the subject 10's motion, and the subject 10 may fall through the gap.

FIGS. 4 and 5 illustrate embodiments related to the second imaging mode of the present invention.

As shown, unlike the first imaging mode, the subject 10 may lie in the width direction in the second imaging mode. In this embodiment, the center table assembly may remain in the reference position without displacement. However, both the first displacement table assembly and the second displacement table assembly may be displaced from both sides. As a result, the first displacement table 310, which was located on the left side in the longitudinal direction in the first imaging mode, may be positioned at a front left side in the width direction, and the second displacement table 410, which was on the right in the first imaging mode, may be positioned at a front right side in the width direction.

This displacement may occur since a first table rotating arm 530 and a second table rotating arm 540, which face the main body 110, may rotate about 180 degrees in the opposite direction. To do so, the second support 343 of the first displacement table assembly may be detached from the first body connector 241 of the center table assembly, and the second support 443 of the second displacement table assembly may be detached from the second body connector 243.

However, in the second imaging mode, a gap may occur between the first displacement table 310 and the second displacement table 410 since the first displacement table 310 and the second displacement table 410 may not be in contact with each other. Therefore, as shown in FIG. 9, a detachable member 444 of the second support 443 of the second displacement table assembly may be coupled to the corresponding mean 347 of the second support 343 of the first displacement table assembly. At the same time, a detachable member 344 of the second support 343 may be coupled to a corresponding mean 447 of the second support 443.

Then, as shown by the arrow in FIG. 5, the arm operating part 120 may be rotated. Through this, 3D imaging of the subject 10 may be obtained. The operator may identify a target area of the subject 10 and obtain a desired 3D image.

FIGS. 6 and 7 relate to the third imaging mode according to a preferred embodiment of the present invention.

In this mode, the subject 10 may not lie on the table but stand on the floor. This imaging method, in which a standing small animal is imaged from the side, is essential for animal diagnosis. In the prior art, separate equipment has been required. In particular, in this imaging mode, the X-ray tube 121 and the X-ray detector 123 must be capable of performing horizontal imaging close to the floor. That is, the arm operating part 120 may be moved to the lowest position level, and the arm rotating part 130 may be moved along the elevating part 113 of the main body 110. In this downwardly displaced state of the arm rotating part 130, as illustrated, the X-ray tube 121 may irradiate the subject 10 with X-rays.

To implement the imaging mode, the center table assembly and the displacement table assemblies described above must be located outside the rotational radius of the arm operating part 120 and must not be present in the alignment line of the X-ray tube 121 and the X-ray detector 123.

The first body connector 241, which may be coupled to the main body 110 of the center table assembly, may be detached from the main body. Then, a rotating hinge 235 on a lower end of a second vertical support 223 may be eccentrically rotated about the second vertical support 223 in a counterclockwise direction, thereby allowing the center table assembly to be separated to the farthest extent from the main body 110, as shown. Accordingly, the first displacement table assembly, which is connected to the center table assembly, may also be moved together. Meanwhile, the second displacement table assembly may be separated from the center table assembly and moved to a separate position.

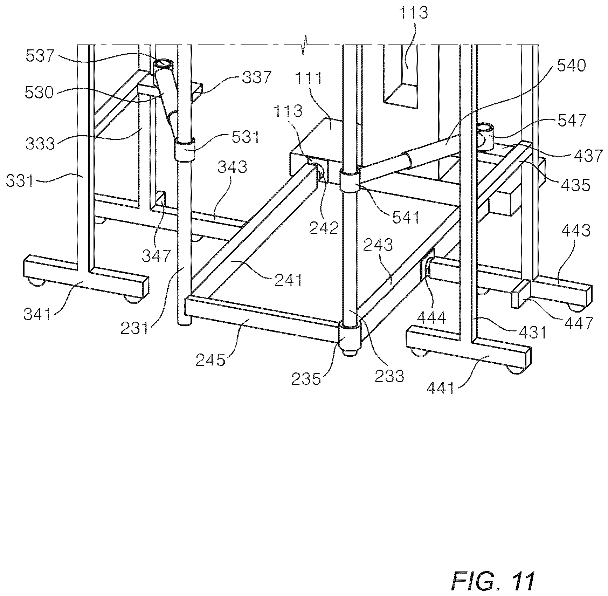

FIGS. 11 to 13 illustrate configurations below the table assemblies of a preferred embodiment of the present invention once again in detail.

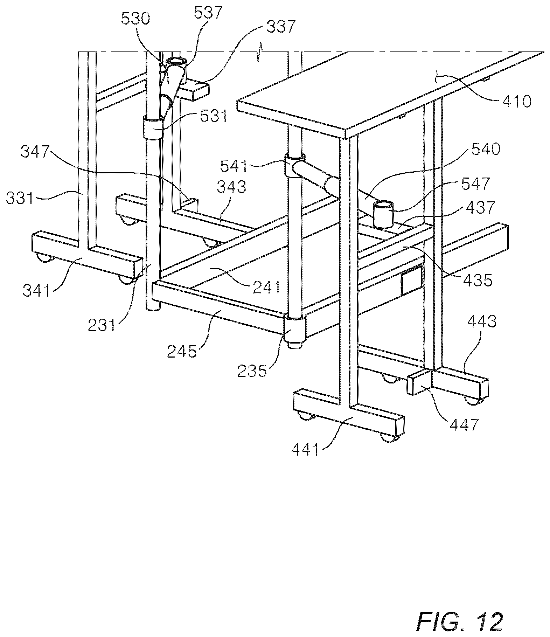

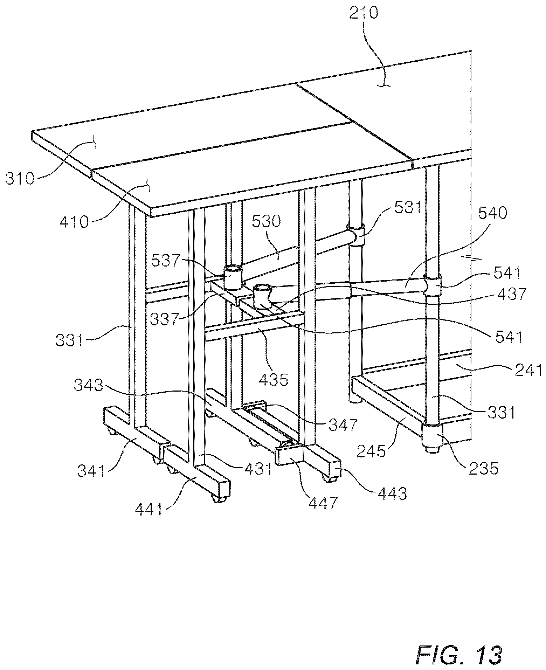

FIG. 11 shows the configurations under the table of the X-ray imaging apparatus for animals in the first imaging mode. In this state, the detachable components are as follows:
(1) The first body connector 241 may be separated from a lower portion 111 of the main body. In the first and second imaging modes, a detachable member 242 may be coupled to the lower portion 111 of the main body. In the third imaging mode, the first body connector 241 may be separated from the lower portion 111 of the main body by releasing the coupling between the detachable member 242 and the lower portion 111 of the main body.
(2) In the first imaging mode, the second support 443 of the second displacement table assembly may be integrally coupled to the side surface of the second body connector 243 through a detachable member 444. However, in the second imaging mode, the second support 443 of the second displacement table assembly 400 may be separated from the second body connector 243 by releasing a detachable member 444. After detachment, as shown in FIG. 12, the second displacement table assembly may be moved forward in the width direction. The fully displaced state is illustrated in FIG. 13.
   Likewise, the relationship between the first body connector 241 and the second support 343 of the first displacement table assembly 300 may be configured in the same manner as described above for the second body connector 243 and the second support 443 of the second displacement table assembly.
(3) A body of a first table rotating arm 530 and a body of a second table rotating arm 540 may be separable. The first and second table rotating arms 530 and 540 may serve to connect the center table assembly and the displacement table assemblies to each other in the first and second imaging modes. However, as shown in FIGS. 6 and 7, in the third imaging mode, the center table assembly and the displacement table assemblies may not need to be separated from each other. In particular, the second displacement table assembly may be separated from the center table assembly. That is, at least the body of the second table rotating arm 540 may have a separable structure, so that the second displacement table 410 may be separated and isolated from the center table 210 as illustrated in FIGS. 6 and 7.

Referring again to FIGS. 11 to 13, the rotatable components of the table assembly system of the present invention are as follows. In this specification, the rotatable components are provided using hinge mechanisms. First, the rotatable components may operate when switching the system from the first imaging mode to the second imaging mode or vice versa. Hinges are illustrated with reference numerals 531, 537, 541, and 547. In other words, the hinges provided on the table rotating arms 530 and 540 may operate. Additionally, when switching the system from the first imaging mode to the third imaging mode or from the third imaging mode to the first imaging mode, a rotating hinge 235 provided on the lower end of the second vertical support 233.

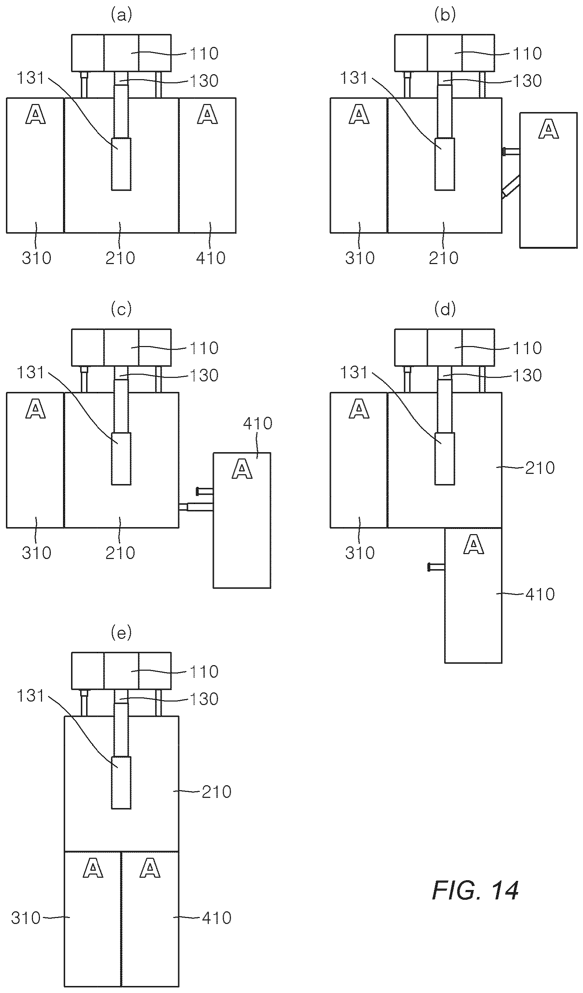

FIG. 14 illustrates a process of performing the table displacement system from the first imaging mode to the second imaging mode according to a preferred embodiment of the present invention. FIG. 14(a) shows the first imaging mode, and FIG. 14(e) shows the second imaging mode. In the first imaging mode, the tables may be arranged in the longitudinal direction, and in the second imaging mode, the tables may be arranged in the width direction.

As shown, in the table displacement system according to the embodiment, the first displacement table 310, which is located at the left side in the first imaging mode, may also be located at the left side in the second imaging mode. Likewise, the second displacement table 410, which is located at the right side in the first imaging mode, may also be located at the right side in the second imaging mode. Just as the appearance of the character "A" does not change when flipped horizontally, the front and rear orientation of the displacement tables 310 and 410 may remain unchanged in the embodiment of FIG. 14. This result may be attributed to the operation of the table rotating arms 530 and 540.

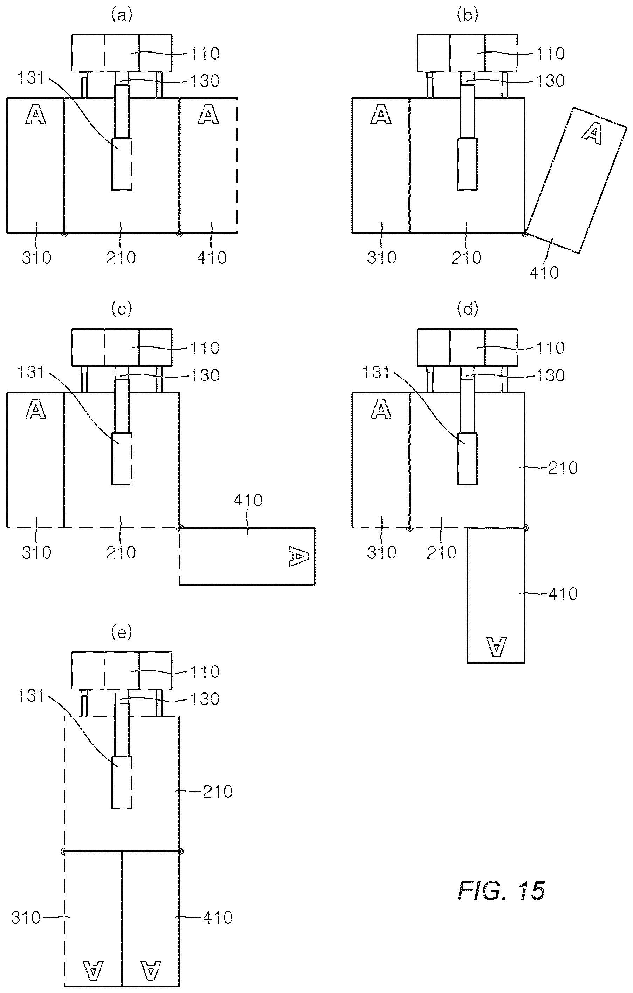

FIG. 15 may illustrate a process of performing the table displacement system from the first imaging mode to the second imaging mode according to another preferred embodiment of the present invention. FIG. 15(a) shows the first imaging mode, and FIG. 15(e) shows the second imaging mode. In the first imaging mode, the tables may be arranged in the longitudinal direction, and in the second imaging mode, the tables may be arranged in the width direction. This result may be the same as that of the embodiment shown in FIG. 14.

As shown, in the table displacement system of this embodiment, the first displacement table 310, which may be located at the left side in the first imaging mode, may also be located at the left side in the second imaging mode. Likewise, the second displacement table 410, which is located at the right side in the first imaging mode, may also be located at the right side in the second imaging mode. In contrast, in the embodiment of FIG. 15, the displacement tables 310 and 410 may be flipped in the vertical direction, like flipping the character "A" upside down, resulting in the front and rear sides being reversed. To implement such an embodiment, pivot hinges may be provided on the corner ends where the center table 210 and the displacement tables 310 and 410 meet, as shown in FIG. 15(a).

In the present invention, various rotating means and coupling means may be disclosed in the center table assembly 200, the first displacement table assembly 300, and the second displacement table assembly 400. The rotating components may be well-known elements and may be variously substituted according to design requirements and field needs, and such substitutions may be well known to those skilled in the art. Therefore, for example, the configuration described as a hinge may not be limited to a specific hinge component.

Regarding the specifications of the present invention, the center table 210 may be designed to have a length of about 700 mm and a width of about 00 mm. The displacement tables 310 and 410 may be designed as about 350 mm × about 800 mm. In the first imaging mode, the table may have a total length of about 1400 mm. The dimensions may be modified by several tens of centimeters in consideration of the body size of large dog breeds. Such specifications of the X-ray imaging apparatus for animals may not be suitable for human use. However, for small animals such as dogs and cats, the apparatus may be most appropriate. Above all, the present invention emphasizes that, with a single apparatus, under the constraint of limited indoor installation space, all of the following imaging types may be performed at animal hospitals: general imaging for obtaining 2D diagnostic images, stereoscopic imaging for obtaining 3D diagnostic images, and stand imaging performed while the small animal stands on four legs. As described in detail above, this may be achieved by the dynamic system of the center table assembly and the displacement table assemblies on both the left and right sides.

Meanwhile, the scope of protection of the present invention may not be limited to the above-described embodiments and their specific descriptions or expressions. The present invention is not limited by the visible shapes, numerical values, or proportions shown in the drawings, which are provided for ease of explanation. Furthermore, it is to be clearly understood that the scope of protection of the present invention may not be limited by obvious modifications or substitutions within the technical field to which the invention pertains.

## Claims

1. An X-ray imaging apparatus for animals comprising:
a main body;
an arm operating part structured such that an X-ray tube and an X-ray detector are arranged in a line facing each other;
an arm rotating part configured to connect the arm operating part to the main body and to rotate; and
a table connected to the main body and on which a subject is to be positioned, the table comprising a center table assembly and displacement table assemblies connected to both sides of the center table assembly, respectively,
wherein, in a first imaging mode, the displacement table assemblies are located on left and right sides in a longitudinal direction of the center table assembly, respectively, so as to secure a reference position for an operator, and
in a second imaging mode, a first table rotating means, which connects the center table assembly and a first displacement table assembly of the displacement table assemblies, operates to move the first displacement table assembly to a front-left side in a width direction of the center table assembly, and a second table rotating means, which connects the center table assembly and a second displacement table assembly of the displacement table assemblies, operates to move the second displacement table assembly to a front-right side in the width direction of the center table assembly, such that rotation of the arm operating part is ensured.

2. An X-ray imaging apparatus for animals comprising:
a main body;
an arm operating part structured such that an X-ray tube and an X-ray detector are arranged in a line facing each other;
an arm rotating part configured to connect the arm operating part to the main body and to rotate; and
a table connected to the main body and on which a subject is to be positioned, the table comprising a center table assembly and displacement table assemblies connected to both sides of the center table assembly, respectively,
wherein, in a first imaging mode, the displacement table assemblies are located on left and right sides in a longitudinal direction of the center table assembly, respectively, so as to secure a reference position for an operator, and
in a second imaging mode, a first table rotating means, which connects the center table assembly and a first displacement table assembly of the displacement table assemblies, operates to move the first displacement table assembly to a front-left side in a width direction of the center table assembly, and a second table rotating means, which connects the center table assembly and a second displacement table assembly of the displacement table assemblies, operates to move the second displacement table assembly to a front-right side in the width direction of the center table assembly, so as to ensure rotation of the arm operating part, and
in a third imaging mode, the arm rotating part is displaced downward from the main body, and among the connectors connecting the center table assembly to the main body, a connector that is detachably coupled is detached, and the center table assembly is eccentrically rotated via a rotating hinge provided at an end of the other connector, so that the center table assembly is spaced apart from the main body to the maximum extent, thereby ensuring horizontal imaging at a lowest position level by the arm operating part.
